# EUROPEAN PATENT APPLICATION

(11) **EP 3 072 446 A1**
(43) Date of publication of application: **28.09.2016**
(21) Application number: 15305439.0
(22) Date of filing: 26.03.2015
(51) Int. Cl.: A61B 5/16, A61B 5/00

(54) **Mental suffering monitoring system**

(71) Applicant: Digital for Mental Health, 91300 Massy (FR)
(72) Inventor: Fompeyrine, Denis, 91300 Massy (FR)
(74) Representative: Cabinet Plasseraud

(57) **Abstract**

The system comprises:
- a measurement system (23) which measures an index of non-conscious adjustments achieved by a human user, as a composite index of user-specific physiological data and user-specific behavioural data,
- a calculation system (25) calculating a pattern index representative of the location of the user inside a mental suffering grid comprising patterns of mental suffering from a predictive model (16), and pre-established structured experimental data descriptive of relationships between values of index of non-conscious adjustments achieved by experimental human users and said experimental human user's mental suffering historical data.

## Description

### FIELD OF THE INVENTION

The instant invention relates to mental suffering monitoring systems.

### BACKGROUND OF THE INVENTION

With the development of telecommunication systems, there has been a trend for monitoring people by using such systems. Indeed, the more data can be obtained about someone, the more appropriate service can in theory be rendered. A typical example of such monitoring can be found in sales and advertisement, where paper questionnaires about consumption habits were gradually replaced by on-line polls, and even survey of electronic communication.

This trend was also observed in the medical world. In the field of diagnosis, for example, in everyday life, the doctor (or physician) has very little time with the patient in order to observe the patient, and perform a diagnosis based on this information. During the interview, patients do not always express what is necessary for the physician to make the diagnosis in the context of assessment grids of mental illness, such as DSM-V. The duration of the interview counts for a lot, 20 minutes minimum should be required. Otherwise further evolution of the disease can be very rapid out of the consultation. According to a recent study (*"*Clinical diagnosis of depression in primary care: a meta-analysis", Mitchell and al., The Lancet, Volume 374, Issue 9690, 22-28 August 2009, p. 609), 50% of primary care physicians (general practitioners) miss the diagnosis of clinical depression.

A better, and in particular, longer, observation of the patient could be useful in order for the doctor to obtain more information about the patient, so as to be able to perform a proper diagnosis. However, for economical reasons, longer observation periods are not possible. Further, some patients may be unwilling to visit their doctor, whereby a diagnosis is not possible.

The above discussion is particularly true in the field of mental health. For physical illnesses, physiological symptoms can be detected to enable a diagnostic. In the field of mental health, however, there might be few visible symptoms of the illness, which are difficult to detect, and/or can be more or less hidden by the patient, especially for primary care physicians, or even specialists in other fields than mental health. Especially, the patient might visit the doctor about a physical illness which may be caused, or at least superimposed to a mental illness. Not even mentioning being able to diagnose a mental illness, the patient's doctor has to decide whether it is necessary for the patient to visit a specialist based on only a few minutes spent with his patient.

It was proposed, in the art, some expert systems enabling to obtain some data about patients, especially in view of psychological evaluation.

WO 2014/110,120 is an example of such a system. This system is in fact primarily designed as an expert system for any kind of health-related issue (health-related issues associated with food served as a restaurant, with kayaking as an activity, with travelling, ...). The "mood" of the patient is used as one of the factors to be considered by the expert system. By the very end of the document, it is mentioned that the "overall mental state" of the patient can be evaluated by aggregating "sentiments" determined based on emoticons used by the patient in text messages s/he sends. Deterioration of the "mental state" is said to be detectable. Two examples are given, namely:
- "a prolonged period of depression or anger" in a patient without a history of psychiatric illness, and
- "a sudden euphoria" for a patient which has a history of depression.

Hence, this document mentions determining possible mental illness based on a history of emoticons used by the patient in text messages.

EP 2 660 745 pretends being able to achieve a diagnostic of psychiatric disorders such as *"MDE, Manic, Dysthymic, Anorectic, Bulimic, Psychotic, Obsessive-compulsive, Panic, Phobic, GAD, Dissociative, Hypochondriac, PTSD, BLPD, Substance Abuse, Sleep disorders",* based on changes in PC, phone or e-mail activity and using machine-learning. However, there is no practical description of how this could be achieved.

In fact, there are many such documents which mention being able providing a diagnosis, or at least a support to diagnosis, of mental illness from computing patient-specific data. Reference is made for example to WO 2012/025,622, WO 2011/109,716, US 2011/118,555, US 2008/208,015, WO 2006/090,371, to name a few.

If some of these documents go into many details about the data to be processed, there is not much description about the way to effectively diagnose or support diagnosis of a mental illness based on this data.

It appears likely that these systems are unable to do more than count events.

Slightly different is WO 2007/107,900 which discloses a watch enabling to display a level of "coolness" of the wearer based on physiological data. If the wearer does not sweat, or has a low heart rate, it is displayed that s/he is "cool". "Coolness" is mentioned as the ability to cope with stress. However, it is doubtful that this device would enable to distinguish between a wearer sweating and having a high heart rate as a reaction to stress versus as a reaction to doing twenty push-ups. Therefore, this device would be operative only (if at all) when the user knows s/he is experiencing stress.

There therefore remains a need for a system enabling to practically monitor the mental suffering of an user.

### SUMMARY OF THE INVENTION

To this aim, it is provided a human user mental suffering monitoring system.

The user mental suffering monitoring system comprises a measurement system. The measurement system repeatedly measures an index of non-conscious adjustments achieved by a human user.

The measurement system comprises a processor enabled to access a memory storing time-dependent user-specific data sets comprising both time-dependent user-specific physiological data sets comprising data representative of a physiological parameter of the user, and time-dependent user-specific behavioural data sets, comprising data representative of a behavioural parameter of the user.

The processor comprises a calculator repeatedly analyzing said time-dependent user-specific data set to repeatedly determine a value for an index of non-conscious adjustments achieved by the human user from said time-dependent user-specific data sets, as a composite index of user-specific physiological data and user-specific behavioural data.

The user mental suffering monitoring system comprises a calculation system calculating a pattern index, representative of the user's likeliness of exhibiting a pattern of mental suffering, representative of the location of the user inside a mental suffering grid comprising patterns of mental suffering from a predictive model, based at least on the values for the index of non-conscious adjustments measured for the user, and pre-established structured experimental data stored in a memory and descriptive of relationships between values of index of non-conscious adjustments achieved by experimental human users and said experimental human user's mental suffering historical data.

The system provides a pattern index for the user. A pattern index is not a diagnostic, but it is data that a doctor can interpret, along with other data, in order to be able to take a decision. The decision could for example be for the user to continue using the system for some time, or to stop using the system. The decision is not necessarily a medical decision.

In some embodiments, one might also use one or more of the following features :
- the human user mental suffering monitoring system comprises a non-conscious-adjustment-index construction module adapted to construct a user-specific index of non-conscious adjustments based at least on a previous index of non-conscious adjustments for the user, and based on said pre-established structured experimental data;
- the non-conscious-adjustment-index construction module compares the previous index of non-conscious adjustments for the user with said pre-established structured experimental data, and determines a parameter to be incorporated in the composite index;
- the monitoring system comprises a clock stamping measured data with time at which measurement of said measured data is performed, whereby user-specific data comprises composite measured data and time data;
- the monitoring system comprises a positioning system stamping measured data with macroscopic location data at which measurement of said measured data is performed, whereby user-specific data comprises composite measured data and macroscopic location data;
- the measurement system comprises a triggering module adapted to trigger measurement of an index of non-conscious adjustments, the triggering module comprising pre-determined triggering rules determining the triggering of a measurement based on user-specific data;
- the index of non-conscious adjustments is a composite index of user-specific physiological data, of user-specific behavioural data, and of occasional data representative of the occasion at which the user-specific data used in the calculation of the composite index is obtained;
- occasional data comprises one and/or more of time and macroscopic location data;
- the index of non-conscious adjustments is a composite index of user-specific physiological data, of user-specific behavioural data, and of user-specific environmental data, relative to data about the environment of the user;
- the human user mental suffering monitoring system further comprises a connection to a medical facility transferring the pattern index and associated human user identification data to the medical facility, and further comprises a discriminating module adapted to discriminate whether the pattern index is to be transferred to the medical facility, the discriminating module comprising pre-determined transfer rules determining when to transfer pattern index to a medical facility;
- the predictive model is a user-specific predictive model, said pre-established structured experimental data also comprising past user-specific values of index of non-conscious adjustments and said user's mental suffering historical data;
- the predictive model is time-dependent, and is repeatedly enriched by structured experimental data descriptive of relationships between values of index of non-conscious adjustments achieved by human users of the monitoring system and said experimental human user's mental suffering historical data;
- the human user mental suffering monitoring system further comprises a user interface comprising one and/or more of the following features:
   . input device adapted to enable the user to record user specific data,
   . display device adapted to display information to the user.

### BRIEF DESCRIPTION OF THE DRAWINGS

Other characteristics and advantages of the invention will readily appear from the following description of one of its embodiments, provided as a non-limitative example, and of the accompanying drawings.

On the drawings :
- Fig. 1 is a schematic view of a user wearing a portable measurement system;
- Fig. 2 is a schematic overview of the system according to one embodiment;
- Fig. 3 shows a grid usable according to one embodiment, where each column shows a pattern, the lines show various levels (top line: high level, bottom line: low level), and the signs show individuals in the grid.

On the different Figures, the same reference signs designate like or similar elements.

### DETAILED DESCRIPTION

The system is designed to be used by users for which it appears necessary to monitor mental suffering. Monitoring the mental suffering of the user could appear necessary to a doctor, or to the user by himself, for example. A "user" as used herein is not necessarily a patient, in that a user does not necessarily have an already diagnosed mental illness, or even an illness, generally speaking. Primarily, the system seems intended to be used for users which do not have a diagnosed mental illness. The system is for example intended to be used by users which are considered at risk of developing a mental illness. Another primary target of the system appears to be users which have been cured from a mental illness, especially to monitor risks of relapse. The user does not necessarily need to exhibit mental suffering. The system might be used with users which exhibit a risk of mental suffering. Other uses than the above uses appear possible.

Here, the system is described from the user's point-of-view. It should be reminded that this system could alternatively be seen from the service provider's point-of-view, comprising one or more central stations 15, end-user stations 10+23 and medical stations 18 at least, or from other points of view.

The system focuses on the quantification of non-conscious adjustments performed by the user as a key data useful in the evaluation of mental suffering. Such data might be taken into account by a doctor when diagnosing or treating mental health.

The system focuses on the quantification of non-conscious adjustments performed by the user in everyday life.

Non-conscious adjustments include the unconscious efforts done by the user to adapt to the every-day situation s/he has to handle.

### Description of non-conscious adjustments

There may be various causes to the development of mental illness. It goes beyond the present document to go into much details about the mechanisms which cause the development of mental illness. However, it can be said that mental illness may be developed as a consequence of trauma experienced by the person. Trauma can be anything from going to war to missing one's bus, and people react differently thereto.

When affected by trauma, people use various behavioural strategy to cope with everyday's life situation.

Continuing with the example of missing one's bus, such behavioural strategy may include, for some people, one or more of:
- buying a new watch,
- leaving 5 minutes earlier for future appointments,
- checking one's watch more repeatedly before appointments,
- sweating or having increased heart beat before appointments, ....

The above list is far from limiting. Some persons may exhibit one or more of the above behavioural strategies, or even no coping behavioural strategy at all.

These behaviours may also evolve with time. For example, with time, the person would leave less and less early before appointments.

Most of these behavioural strategies can be measured. However, as such, none is indicative of mental suffering. From a mental health perspective, both persons with mental suffering and persons without may implement any of the above behavioural strategies. For example, both persons with/without mental suffering may check their watch more repeatedly before meetings. Alternatively, a person with mental suffering may exhibit none of the above behavioural strategies, and a person without mental suffering might.

Thus, measurement of coping behaviours might not be the main parameter when monitoring status of mental suffering.

An interesting parameter to be used by the doctor or the user appears to be the non-conscious adjustments by the user to adapt with the everyday life situation. As a rather simple example from the above scenario, it may be nothing for the user to leave 5 minutes earlier than usual in order to be on time on future appointments. But for some users, this may require adjustments which require terrible efforts. The present system achieves quantifying these adjustments.

These adjustments might not be detectable when measuring the coping behavioural strategy. Indeed, the user performs some control of his/her coping behavioural strategy. However, there are some non-conscious occurrences of the efforts performed by the user to adjust, and this is what the present system measures. These non-conscious adjustments also include the unconscious efforts made by the user.

### System overview

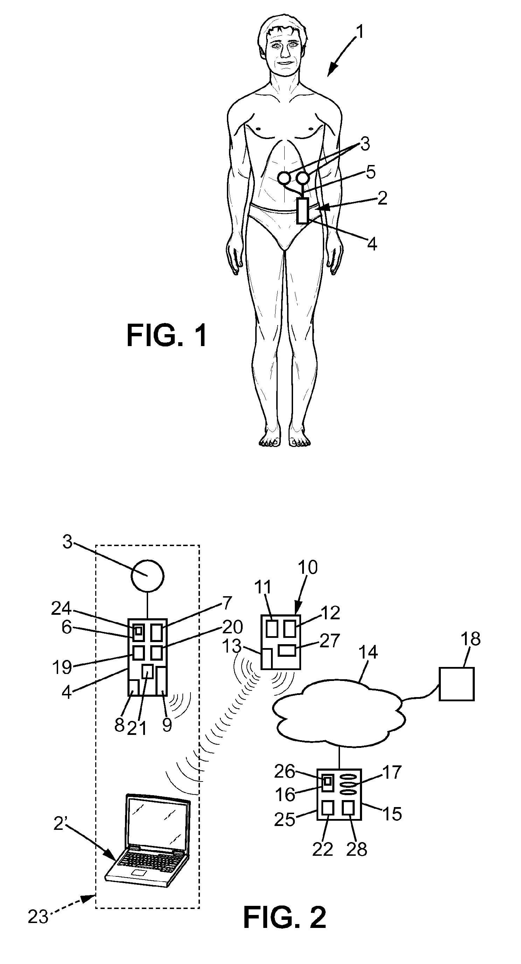

Determining the non-conscious adjustments requires data to be processed. Fig. 1 schematically shows a user 1 of a system. The system comprises an acquisition system 2. The acquisition system 2 is bearable by the user 1. There are many possibilities for making the acquisition system 2 bearable by the user. Fig. 1 is just one schematic example.

The acquisition system 2 comprises an active detection part 3, and an electronic part 4. The active detection part 3 and the electronic part 4 can be connected to one another by any suitable way, for example through a wire 5, or wireless. The electronic part 4, being wearable, and thus still limited in power consumption, can be limited to the basic functions for operating the detection part 3. In particular, it would comprise a processor 6 handling the detection part 3, a memory 7 for storing detected data by the detection part 3, a battery 8 for powering the acquisition system 2, and a communication module 9 for communicating with the outside. The outside is embodied as a base station 10. Such communication can be wireless. Alternatively, the wearable device could be from time to time electrically connected to a base station 10 and communication would take place at that time.

The processor 6 could be programmed to pre-treat data stored in the memory 7, for example by pre-filtering, or to reduce the size of the data sets, in view of future computation or communication.

According to the present example, the acquisition system 2 is shown as two parts communicating with one another through a wire. However, in variant, the acquisition system could be a single device, whereby the electronic part 4 is integrated with the detection part 3 into a single device.

The base station 10 can be permanently connected to a power line. It could comprise a memory 11 for storing data transferred from the acquisition system 2, a processor 12, and a communication module 13 for communicating with the acquisition system 2. The base station 10 is for example permanently at the user's place. The communication module 13 can also be designed to access the outside world, through any suitable network 14, for example through the Internet. In particular, the base station 10 can access a distant server 15 comprising a predictive model 16 and experimental data 17, as will be described in more details below.

The system may further comprise medical stations 18. The medical stations 18 can be connected to the other components of the system through the network 14. The medical stations 18 will be described in more details below.

Back to the user's end, the use of an active detection part 3 was described above. The active detection part 3 may sense one or more parameters. In case of sensing more than one parameter, one could consider a plurality of active detection parts 3, as shown. For example, all detection parts 3 are connected to the same electronic part 4. Alternatively, they could be connected to different electronic parts 4. The system then has plural acquisition systems 2, each connected with the base station 10. According to another alternative, one active detection part 3 could be integrated in the electronic part 4. This could for example be the case when one active detection part 3 is a ground positioning system, sensing its own position.

In general, the base station 10 can be connected to acquisition systems 2. In addition to the wearable acquisition systems 2, the system may comprise some acquisition systems 2 which are usual electronic devices 2' which have been upgraded to act as an acquisition system for the present system. Upgrading could be done, for example, by uploading a specific software to the pre-existing electronic device 2', this software detecting the data which is to be detected on the electronic device, and forwarding it to the base station 10.

If necessary, the base station 10 may comprise an input device, such as a keyboard or the like, enabling the user to record user-specific data. The base stations 10 may also comprise other user interfaces, such as a display 27, so as to allow interactive communication with the user.

### User-specific data and sensors

One or more types of user-specific data can be used within the system. One type includes user-specific physiological data. Another type includes user-specific behavioural data.

Physiological data: These data can include any accessible information about parameters representative of the physical state of the body of the user - body temperature, heart rate, blood pressure, dermal electro-activity, caloric metabolism, breathing rhythm, sweating, swallowing, gnashing of teeth, ...

Behavioural data: These data can include any accessible information about parameters representative of the behavioural activity of the user. One typical example could be the position of the user. Another example, based on the position of the user, could be the instantaneous speed of the user, or its average speed during a pre-determined period of time. Yet another example based on position data is acceleration data.

Another example of behavioural data comprises data based on the use by the user of electronic devices 2'.

If, as described above, when the electronic device is upgraded to serve as an acquisition system, they can provide user-specific behavioural data.

For example, computer use data could be behavioural data. Computer use data can include any kind of data retrievable from the computer, such as for example switching on/off, staying idle, used program, typed text, ... .

Communication system use data is another example of behavioural data. Communication system use data can include any kind of data retrievable from a communication system (phone, ...), such as for example switching on/off, staying idle, identity of communicating party, nature of communication (text, sound, e-mail, short messages, ...), ....

Other examples are possible. If the fridge door has been upgraded as an acquisition system, it can provide data as to the user opening/closing the fridge. Typical electronic devices 2' may include computers, TV, camera, electronic accesses and door locks, kitchen appliances, heating system, or connected medical devices (connected inhalation spray, or the like)....

Another example of behavioural data includes active behavioural data. Active behavioural data includes data which the user provides when actively prompted by the system. One typical example is data extracted from forms sent to the user by the system and filled by the user.

Optionally, one may also use environmental data. Environmental data relates to data about the environment of the user. Typical environmental data may include sound vibration, brightness, ambient temperature, electronic transmissions, ....

### Data acquisition

Data acquisition is repeated in time. For example, data acquisition is performed at a given time frequency. The frequency may depend on the sensor. A "data set" corresponds to stored data in a certain occasion. Since more and more data is acquired along time, a data set may grow with time. Alternatively, the volume of the data set may remain stable between bounds, when older or irrelevant measured data is discarded. Various embodiments are possible. A data set may comprise sub-data sets. For example, a sub-data set could be defined for some periods of acquisition, for a sub-set of acquisition systems, ....

### Composite data

Composite data is used to designate data which comprises both physiological data and behavioural data.

For example, composite data may comprise data for one or more physiological parameter, and one or more behavioural parameter.

Such composite data may be either combined or juxtaposed.

For example, juxtaposed composite data could be stored in multi-dimensional vector form. X= (Aᵢ, Bⱼ, Cₖ) may represent a vector comprising data for three different parameters A, B, C, where i, j, k indicate one or more values for the parameters A, B, C, respectively.

Combined composite data could be obtained by a pre-defined operation f. For example X=f (Aᵢ, Bⱼ, Cₖ) may represent a combined composite data obtained by applying a pre-defined function f to three different parameters A, B, C.

### Compositing data with time t

As discussed above, most physiological and behavioural data is measured at a given time. The electronic part 4 may comprise a clock 19 enabling to composite acquired data with time. Stored data is then a composite of the measured data itself, and time at which it is measured. If the measured data can be acquired only during a long time, rules might be set to determine which is the time which corresponds to the recorded data. For example, if the behavioural data is the extent of a movement by the user, with this movement taking minutes or hours, the composited time might be that of the beginning of the movement, the end of the movement, or an average between these two times. Other examples are possible.

By using a clock, duration data itself could also be a user-specific data. If an acquisition system provides some data composited with time, the user-specific data could be for example the duration of a given phenomenon. Typical example could include duration of the time during which the user's heart rate is over a pre-defined threshold, duration of user walk (time spent with a movement comprised between 1 and 5 meters/s), ...

### Compositing data with macroscopic location Z

As discussed above, most physiological and behavioural data is measured when the user is in a given macroscopic location. The electronic part 4 may comprise a ground positioning system 20 enabling to composite acquired data with macroscopic location. Stored data is then a composite of the measured data itself, and macroscopic location at which it is measured.

Macroscopic location can be considered as structured location data from the ground positioning system. Structured location data could be defined as locations where the user spends most of its time. For example, the world could be dealt in three different macroscopic locations, namely "home", "office", "rest of the world". These macroscopic locations could be defined for the user when setting the system. Macroscopic locations differ from the location data provided by the ground position system by their sociological definition. Macroscopic location can thus be composited with location data, even though both are obtained from the ground positioning system, as can be seen from the examples below.

An example of a composite index of physiological and behavioural data could for example be transcripted as : "the user moves a lot and has high blood pressure".

An example of a composite index of physiological and behavioural data and time could for example be transcripted as : "the user moves a lot and has high blood pressure at nights".

An example of a composite index of physiological and behavioural data and macroscopic location could for example be transcripted as : "the user moves a lot and has high blood pressure at the office".

An example of a composite index of physiological and behavioural data, macroscopic location and time could for example be transcripted as : "the user moves a lot and has high blood pressure at the office at nights".

### Occasional data

Occasional data will be based on interpreted time and/or location data obtained from clock and/or positioning system, respectively. The measuring system may comprise a time interpretation module adapted to convert time data into interpreted time data. Interpreted time data would correspond to time periods having a social meaning. Such time periods might include, for example: day-time, nighttime, morning, afternoon, evening, ordinary weekday, Wednesday, Saturday, Sunday, seasons, .... Indeed, interpreted time might have a specific meaning when interpreting measured data. A data measurement may have a quite different meaning depending on the macroscopic time, or occasion, for which it is measured. For example, leaving home at 3 AM or at 5 AM differs by two hours, such as leaving home at 5 AM or at 7 AM. However, leaving home at 7 AM might be indicative of a quite different behaviour than leaving home at 5 AM, whereas leaving home at either 3 AM or 5 AM might not be so indicative of different behaviours. Using interpreted time data may enable to discriminate the events as leaving home "at night" vs "in the morning". Time interpretation rules might be stored in the memory 7.

The same applies with macroscopic location data. Interpreted location data may include various predetermined macroscopic location, such as "at home", "at the office", .... Interpreted time and/or location is designated as "occasional data", representative of the occasion at which the user-specific data is obtained.

### Measurement of the composite index

The composite index will be measured through calculation of user-specific data.

The processor 6 comprises a calculator 24 which calculates a value for the composite index. As explained above, the calculated composite index is a composite index of behavioural data and physiological data.

For example, the composite index is defined according to a rule stored in the memory 7. This rule can comprise the parameters A, B, C to be considered within the composite index, as well as the function f, if any.

The occurrences of calculation of the composite index can be predetermined.

For example, a value of the composite index is calculated at a given frequency, as monitored by the clock 19.

According to another example, calculation of a value of the composite index is triggered. Triggering can be caused by an external command, coming for example through the network 14.

Triggering can also be caused by analysis of the data.

The electronic part 4 may include a triggering module 21 which will trigger measurement of the composite index. The triggering module 21 comprises pre-determined triggering rules determining the triggering of a composite index measurement based on user-specific data.

The triggering rule might generally be a simpler rule than rules used for the calculation of values for the composite index. The triggering rule might for example be based on a single parameter, or on a few parameters. The triggering rule might for example cause triggering when a monitored parameter exceeds a pre-defined threshold. Examples of triggering rules comprise, for example, a sudden movement by the user, a long time with no movement by the user, an amplitude of a movement of a user, a value of a physiological parameter exceeding a pre-defined threshold, .... A simple rule requires less calculation, and can be assessed by the processor 6 of the portable electronic part 4 with little power consumption.

When the triggering rule triggers measurement of the composite index, the value for the composite index is calculated. This value is for example calculated by the processor 6 and stored in the memory 7. This calculation is performed only upon triggering, which saves computing cost. Indeed, if the composite index is complex, and that its calculation requires to handle a large volume of data, this calculation is performed only from time to time, and when it is pre-determined that it might be useful.

The value for the composite index can be transferred to the base station 10 and stored in the memory 11. In particular, the following can be transferred to the memory 11 of the base station 10:
- the composite index (i.e. juxtaposed or function f),
- the value of the composite index (which cn be a vector),
- a time for which the value of the composite index was calculated,
- a macroscopic location for which the value of the composite index was calculated.

The measured composite index is an indicative of the non-conscious adjustments made by the user.

### Pre-established structured experimental data

The server 15 houses a calculation system 25 calculating a pattern index for the user. The calculation system 25 comprises a processor 22 for doing such calculation.

A pattern would be a feature which may be present for someone which suffers of a given illness. However, it should be noted some people may suffer from this illness while not experiencing this feature. Further, some sane person may show features which are close to a pattern while still not suffering from an illness.

The processor 22 also has access to pre-established structured experimental data. The pre-established structured experimental data is contained in a database 17. These data are pre-established, with respect to the time at which the processor 22 accesses them. They are experimental because they are, at least partly, based on previous observations of the user and/or of people. They are structured because they enable to retrieve some information.

The pre-established structured experimental data for example comprise a set of data allowing to determine a pattern index for the user.

For this, the pre-established structured experimental data are descriptive of relationships between values of index of non-conscious adjustments achieved by experimental human users, and the respective mental suffering historical data of these users. In particular, for users having a non-zero mental suffering historical data, the pre-established structured experimental data comprises data related to patterns of mental suffering for the person, and values of associated composite index for this person.

Indeed, patients suffering of mental illness exhibit some specific patterns of the illness. It is also believed that people on the verge of suffering from a mental illness, and people having recovered from mental illness exhibit these patterns. There are many mental illnesses and, for a given mental illness, there are many various patterns that can be present, and which will differ from person to person subject to a given mental illness. A given patient is likely to exhibit at the same time various patterns.

In the pre-established structured experimental data, data can be structured as a grid listing patterns of mental suffering, and the associated people may be registered in this grid. For each pattern, people may be registered at one level of mental suffering associated with this pattern.

### Predictive model

The predictive model is adapted to locate the user in the mental suffering grid, based on the composite index measured for the user, and on the pre-established structured experimental data. In particular, the predictive model compares the measured composite index for the user and the pre-established structured experimental data. In particular, the predictive model may look for experimental data showing similar values of composite index as the user. Hence, the predictive model may enable to select experimental data showing composite index "close to" the composite index measured for the user. "Close to" or "similar" can be estimated according to various methods, as the calculation of a distance in multi-dimensional environments. Among the close experimental data, the predictive model will check for the presence of patterns of mental suffering.

If close experimental data comprises patterns of mental suffering, the predictive model could determine a pattern index for the user, representative of a possible location of the user in the mental suffering grid comprising such patterns.

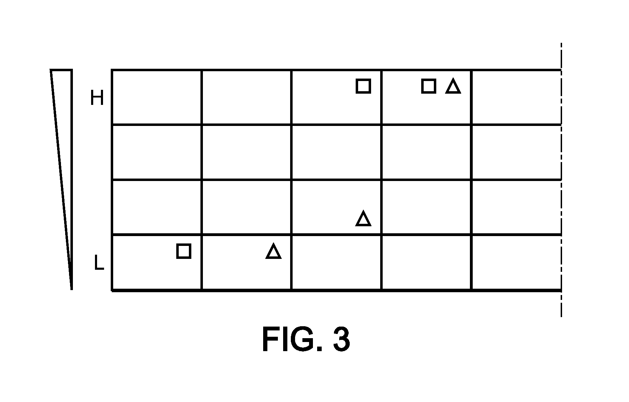

Fig. 3 shows a grid usable according to one embodiment, where each column shows a pattern, the lines show various levels (top line: high level H, bottom line: low level L), and the signs show individuals in the grid. Hence, for the user of the system, it has been determined that s/he was close to experimental data of a person identified as a triangle, and to experimental data of a person identified as a square. Other experimental data, too different from the user, was not shown on this grid.

The pattern index can be any index representative of the possible location of the user in the grid. For example, it may include the maximum, or the average, of the persons remained in the grid, for each pattern, or even the maximum retained pattern. Many approaches are possible. The pattern index calculated for the user may be stored in a memory of the server 15, along with the user identification.

### Non-conscious-adjustment-index construction module

As discussed above, the value of the composite index measured for the user enables to determine similar experimental data. It is however possible that the similar experimental data does not enable by itself to calculate the pattern index. This would be the case when for example, experimental data comprises a wide variety of results with a similar value of the composite index. The experimental data may comprise individuals with different patterns of mental illness.

This could be the case for example in Figure 3, if the square user has experienced some pattern of mental illness that the triangle user has not.

The predictive model comprises a non-conscious-adjustment-index construction module 26. The index construction module constructs a composite index to be measured for the user. Thus, the composite index is a user-specific composite index. In particular, the index construction module constructs a composite index to be measured for the user which is more likely to enable calculating a pattern index.

The construction module will analyse the close experimental data, determined as described above. The construction module will identify, inside the close experimental data, an updated composite index enabling to calculate a pattern index. For example, the identified updated composite index will enable to distinguish, among the close experimental data, individuals with non-zero mental suffering historical data from individuals with zero mental suffering historical data. The identified updated composite index will for example be based on the initial composite index, for example using a different rule taking into account the same user-specific data, or a rule taking into account a new user-specific parameter in addition to the one used to calculate the initial composite index. The identified updated composite index may even discard some of the parameters used when calculating the initial composite index.

For example, on Figure 3, it can be seen that the square and triangle users can be distinguished through their level with respect to the third pattern. Positioning the user with respect to this pattern therefore appears relevant to check whether s/he is closer to the square or triangle user. The construction module accesses the memory 17 to determine, based on composite index measured for the experimental data, the updated composite index which is most suitable for the user to be a relevant composite index.

The updated composite index can be communicated back to the acquisition system 2. Based on the experimental data, it is known that the updated composite index is likely to be indicative of the non-conscious adjustments made by the user. Indeed, it is a parameter which is deemed to be potentially relevant to assess non-conscious adjustments of the user, based on the experimental data.

Either enough data is stored in the memory 7 in order to calculate the updated composite index, or the definition of the updated composite index is stored in order to measure the value for the updated composite index at the next triggering.

### Handling of composite index

The measured composite index may be used in many ways. For example, the measured composite index can be sent to the user. For example, the base station 10 and/or the electronic part 4 may comprise a display 27 displaying the composite index, as well as a value for the measure of the composite index.

For example, the measured composite index can be sent to a medical facility 18. This can be done through the network 14. The calculation system 25 may comprise a discriminating module 28 determining which data is to be sent to a medical facility 18. For example, the data to be sent to the medical facility 18 may include user identification and composite index and/or pattern index and its/their value(s) for this user.

Data received at the medical facility 18 may help the doctor recognize patterns of mental suffering for the user. The doctor can decide to implement actions with respect to the user. These actions might use some features of the monitoring system. For example, the processor 6 or 12 might run a specific computer program designed for the user. Examples of such specific programs include games or questionnaires to be used by the user. User interfaces of the monitoring system, or other user interfaces, might be used by the user to interact with the computer program.

Associated user-specific data and user pattern of mental suffering identified by the doctor can in turn be communicated to the memory 17, so as to enhance pre-established structured experimental data stored there.

The predictive model 16 thus becomes a user-specific predictive model, in that it accesses user-specific experimental data stored in the memory 17.

As it will be understood, the predictive model is also time-dependent, since, with time, it will change, integrating data from various users. This in turn would enable to establish a more precise predictive model.

### Example 1

A predetermined parameter corresponds to a composite index of three variables. A first variable is a behavioural variable, whereas the second and third variables are physiological variables.

An example of the first variable is duration of time spent outside of home.

The measurement system comprises, as a sensor, a positioning system providing position data and a clock. The calculator 24 repeatedly receives position data. The calculator 24 compares position data with "home position data" for the user. "Home position data" for the user may have been pre-defined by the user at set-up of the system, or maybe extracted from the measured position data by the calculator itself according to a pre-defined rule, for example position data provided for most nights [The calculation system would be programmed to assume that the user spends most nights at home]. By comparing position data with "home position data", the calculator 24 identifies that the user leaves or enters home, and registers the corresponding times from the clock. The calculator 24 calculates duration of time spent outside home, for example during the last 24 hours. This measurement is performed every hour.

An example of the second variable is change of weight. The measurement system comprises, as a sensor, a weighing device connected to the calculator 24. The calculator can record measured weights and quantify changes in the measured weight. This measurement is performed each time the user weighs.

An example of the third variable is average swaggering when waking-up. The measurement system comprises, as a sensor, an accelerometer worn by the user, and the clock. The sensor first determines waking-up as a sudden change of position from the lying position to a standing position after a long time in the lying position. The clock might also be used to discriminate whether the user is waking-up. The sensor determines swaggering as a certain recognizable movement of the user at that time. The calculator 24 averages the recorded amplitude of this movement during the last three days, for example. This measurement is performed every day.

The calculator calculates a composite index representative of the non-conscious adjustments made by the user, as a predetermined combination of the first, second and third input data. This provides a value of this composite index. This is performed for example once a day. This is performed for example every day at noon, as triggered by the clock, or every day at waking up, when waking up is detected as a part of detecting the third parameter. When triggered, the composite index measurement would take into account the 24 last values for the first variable, the latest available value for the second parameter (this might be several days old), and the last value for the third parameter.

The calculator 24 determines a value for the composite index of these three variables. This is sent to the calculation system 25. The calculation system compares the measured value for the composite index with pre-established structured experimental data.

For example, the pre-established structured experimental data may comprise information that people with little time spent outside from home, rapid loss of weight, and large swaggering at wake-up are more likely to exhibit some patterns of mental suffering a few days after this composite index has taken a given value.

The predictive model 16 thus enables to compare the measured value of the composite index for the user with other data in order to determine actions to be launched.

Other data can include values of the composite index measured for a representative set of people. The representative set of people can include only sane people. "Sane" people for example include only people with no past known record of mental suffering or mental illness such as depression. Otherwise, or in addition, the representative set of people may include both people with past record of mental suffering and people with no past record of mental suffering. Alternatively or in addition, other data can comprise data from the user himself.

The comparison is programmed so that, for some given result of the comparison, some action can be generated, whereas for some other given result of the comparison, some other action is generated, or no action is generated.

An action might for example be to update the composite index. The predictive model 16 would retrieve information that, for the relevant population (people exhibiting similar values of the initial composite index), heart rate at nights is a relevant parameter to be taken into account in order to discriminate people with no history of mental suffering from others. The server 15 will send back the updated composite index to be determined to the measurement system 2. If heart rate data for the time when the initial composite index was measured is still available, the updated composite index might be measured from the past data. Otherwise, the updated composite index will be measured from now on, according to the above-described scheme.

Updating the composite index can be performed one or more additional times if necessary. If a composite index, or an updated composite index shows that there is little risk for the user to exhibit mental suffering, the composite index will stop being updated. The composite index may stop being submitted to updating after a pre-defined number of updates.

Another action might be to warn the medical facility 18 that the user shows some risks of exhibiting mental suffering associated with some patterns.

### Example 2

According to an example, the system tracks a non-conscious adjustment made by the user when leaving his home.

As a specific example, the behavioural variable is a rate of reproducibility of a given movement.

The physiological variable is a duration during which a physiological data exceeds a peak value during the two hours before performing the given movement.

A trigger of the measurement is the user leaving home. This could be determined simply by the user inputting to the device that he is leaving home (active input action). Alternatively, this could be achieved from detection of measured data. The calculator 24 repeatedly receives position data. The calculator 24 compares position data with "home position data" for the user. "Home position data" for the user may have been pre-defined by the user at set-up of the system, or maybe extracted from the measured position data by the calculator 24 itself according to a pre-defined rule, for example position data provided for most nights [The calculator 24 would be programmed to assume that the user spends most nights at home]. By comparing position data with "home position data", the calculator 24 identifies that the user leaves home.

The calculator 24 calculates a rate of reproducibility of position data prior to the measurement-triggering event from the various position data sets (either they are very different, and the rate of reproducibility is low, or they are very similar, and the rate of reproducibility is high).

A second input data is for example a peak of cardiac activity before the measurement-triggering event. The measurement system 23 repeatedly provides heart rate data. It also includes the clock. The calculator 24 processes the heart data to determine the duration, during a given period before the measurement-triggering event, by which the heart rate is over a given threshold.

The calculator repeatedly calculates a value of the composite index for the user. The calculation system 25 compares the calculated value for the composite index with pre-determined rules generating some specific actions for certain values of the composite index.

The calculator 24 calculates a composite index representative of the non-conscious adjustments, as a predetermined combination of the first and second input data. This provides a value of this composite index on this scale.

The predictive model 16 enables to compare the measured value of the composite index with other data in order to determine actions to be launched.

Other data can include values of the composite index measured for a representative set of people. The representative set of people can include only sane people. "Sane" people for example include only people with no past known record of mental suffering or mental illness such as depression. Otherwise, the representative set of people may include both people with past record of mental suffering and people with no past record of mental suffering. Alternatively or in addition, other data can comprise data from the user himself.

The calculation system 25 retrieves from the experimental data recorded patterns of mental suffering for users which exhibit similar values for the composite index.

If necessary, updating of the composite index might be performed as explained above in relation to Example 1.

### Variants

A given example has been given above.

It is known that computerized systems can be distributed over a network. In the above example, a given distribution of computing power is provided. However, other distributions are possible, depending on the requirements of volume of data to be transferred, energy consumption, computing power, access to external resources, etc.... Networks can be any kind of known networks, and connection between devices of the system could be according to any suitable communication scheme, embodied through wires or wireless.

For example, according to one variant, there is no base station, and the functions of the base station are incorporated in the electronic part. Yet further, the calculation system might be incorporated in the electronic part as well.

The invention sought to be protected is presented as a system. The applicant reserves any right to protect any inventive individual component of the system by any available legal means.

## Claims

1. Human user mental suffering monitoring system comprising:
- a measurement system (23) for repeatedly measuring an index of non-conscious adjustments achieved by a human user, the measurement system comprising a processor (6) enabled to access a memory (7) storing time-dependent user-specific data sets comprising both time-dependent user-specific physiological data sets comprising data representative of a physiological parameter of the user, and time-dependent user-specific behavioural data sets, comprising data representative of a behavioural parameter of the user,
the processor (6) comprising a calculator (24) designed to repeatedly analyze said time-dependent user-specific data set to repeatedly determine a value for an index of non-conscious adjustments achieved by the human user from said time-dependent user-specific data sets as a composite index of user-specific physiological data and user-specific behavioural data,
- a calculation system (25) calculating a pattern index, representative of the user's likeliness of exhibiting a pattern of mental suffering, representative of the location of the user inside a mental suffering grid comprising patterns of mental suffering from a predictive model (16), based at least on the values for the index of non-conscious adjustments measured for the user, and pre-established structured experimental data stored in a memory (17) and descriptive of relationships between values of index of non-conscious adjustments achieved by experimental human users and said experimental human user's mental suffering historical data.

2. Human user mental suffering monitoring system according to claim 1, comprising an non-conscious-adjustment-index construction module adapted to construct a user-specific index of non-conscious adjustments based at least on a previous index of non-conscious adjustments for the user, and based on said pre-established structured experimental data.

3. Human user mental suffering monitoring system according to claim 2, wherein the non-conscious-adjustment-index construction module compares the previous index of non-conscious adjustments for the user with said pre-established structured experimental data, and determines a parameter to be incorporated in the composite index.

4. Human user mental suffering monitoring system according to any of claims 1 to 3, wherein the monitoring system comprises a clock (19) stamping measured data with time at which measurement of said measured data is performed, whereby user-specific data comprises composite measured data and time data.

5. Human user mental suffering monitoring system according to any of claims 1 to 4, wherein the monitoring system comprises a positioning system (20) stamping measured data with macroscopic location data at which measurement of said measured data is performed, whereby user-specific data comprises composite measured data and macroscopic location data.

6. Human user mental suffering monitoring system according to any of claims 1 to 5, wherein the measurement system comprises a triggering module (21) adapted to trigger measurement of an index of non-conscious adjustments, the triggering module comprising pre-determined triggering rules determining the triggering of a measurement based on user-specific data.

7. Human user mental suffering monitoring system according to any of claims 1 to 6, wherein the index of non-conscious adjustments is a composite index of user-specific physiological data, of user-specific behavioural data, and of occasional data representative of the occasion at which the user-specific data used in the calculation of the composite index is obtained.

8. Human user mental suffering monitoring system according to claim 7, wherein occasional data comprises one and/or more of time and macroscopic location data.

9. Human user mental suffering monitoring system according to any of claims 1 to 8, wherein the index of non-conscious adjustments is a composite index of user-specific physiological data, of user-specific behavioural data, and of user-specific environmental data, relative to data about the environment of the user.

10. Human user mental suffering monitoring system according to any of claims 1 to 9, further comprising a connection to a medical facility transferring the pattern index and associated human user identification data to the medical facility, and further comprising a discriminating module adapted to discriminate whether the pattern index is to be transferred to the medical facility, the discriminating module comprising pre-determined transfer rules determining when to transfer pattern index to a medical facility.

11. Human user mental suffering monitoring system according to any of claims 1 to 10, wherein the predictive model is a user-specific predictive model, said pre-established structured experimental data also comprising past user-specific values of index of non-conscious adjustments and said user's mental suffering historical data.

12. Human user mental suffering monitoring system according to any of claims 1 to 11, wherein the predictive model is time-dependent, and is repeatedly enriched by structured experimental data descriptive of relationships between values of index of non-conscious adjustments achieved by human users of the monitoring system and said experimental human user's mental suffering historical data.

13. Human user mental suffering monitoring system according to any of claims 1 to 12, further comprising a user interface comprising one and/or more of the following features:
- input device adapted to enable the user to record user specific data,
- display device (27) adapted to display information to the user.
